# EUROPÄISCHE PATENTSCHRIFT

(11) **EP 0 815 444 B1**
(45) Veröffentlichungstag und Bekanntmachung des Hinweises auf die Patenterteilung: **09.02.2005**
(21) Anmeldenummer: 96902998.2
(22) Anmeldetag: 24.02.1996
(51) Int. Cl.: G01N 31/22, G01N 33/84

(54) **VERFAHREN UND MITTEL ZUR BESTIMMUNG VON JODID**
METHOD AND MEANS FOR THE DETERMINATION OF IODIDE
PROCEDE ET MOYEN POUR LA DETERMINATION D'IODIDE

(30) Priorität: 04.03.1995 DE 19507685
(43) Veröffentlichungstag der Anmeldung: 07.01.1998
(73) Patentinhaber: MERCK PATENT GmbH, 64293 Darmstadt (DE)
(72) Erfinder: FISCHER, Wolfgang, D-64283 Darmstadt (DE); GROH, Thomas, D-65451 Kelsterbach (DE); BEIL, Stefanie, D-64546 Mörfelden (DE)
(86) Internationale Anmeldenummer: PCT/EP1996/000774
(87) Internationale Veröffentlichungsnummer: WO 1996/027794

(56) Entgegenhaltungen:
- EP-A- 0 322 631
- WO-A-92/22806
- ANALYST, Bd. 113, 1988, Seiten 1477-1479, XP002007650 D. M. DAVIES ET AL.: "Determination of peracids in the presence of a large excess of hydrogen peroxide using a rapid and convenient spectrophotometric method"

## Beschreibung

Die Erfindung betrifft Verfahren und Mittel zur halbquantitativen Bestimmung von Jodid in wäßrigen Lösungen, vorzugsweise im Harn.

In der klinischen Diagnostik spielt die Jodidbestimmung im Ham für die Kontrolle der Funktionsfähigkeit der Schilddrüse eine wichtige Rolle. Auch die Bestimmung von Jodid in Lebens- und Futtermitteln gewinnt zunehmend an Bedeutung. Geeignete Schnellmethoden, die routinemäßig eingesetzt werden können, sind für die Jodidbestimmung im Ham bisher nicht beschrieben worden.

Bei den meisten bekannten Verfahren wird eine Vorbehandlung des Hams entweder durch Behandlung mit einer starken Säure oder durch Veraschung bei hohen Temperaturen vorgenommen. Daran schließt sich die Jodidbestimmung an durch Messung der Reduktion von Cer(IV)-Ionen zu Cer(III)-Ionen durch die katalytische Wirkung des Jodids (Sandell-Kolthoff-Reaktion). Die Vorbehandlung mit starken Säuren (Kaliumchlorat in 70 %iger Perchlorsäure) oder die trockene Veraschung des Urins mit Kaliumcarbonat dient der Entfernung von Substanzen, die bei der Reduktion der Cerionen stören können. Dabei entstehen toxische Perchlorsäuredämpfe und die ebenfalls entstehenden Perchlorate sind bekanntlich explosiv.

In Anal. Chim. Acta 282, 87 (1993) ist ein Verfahren zur Bestimmung von Jodid mit Chlorpromazin und Wasserstoffperoxid beschrieben, das jedoch sehr störanfällig für viele Substanzen ist, die vor allem im Ham sowie in Lebens- und Futtermitteln vorkommen. Weiterhin ist aus der DE-OS 37 43 224 ein Verfahren zur Bestimmung von Persäuren bekannt, indem man zur Probelösung ein Chromogen und Jodid hinzugibt. Der Fachmann konnte aus dieser Publikation jedoch nicht entnehmen, daß diese Reaktion zum Nachweis von Jodid eingesetzt werden kann.

Der Erfindung liegt die Aufgabe zugrunde, einen einfachen und schnellen Test zur Bestimmung von Jodid zur Verfügung zu stellen, mit dem auch ungeschultes Personal problemlos selbst geringe Mengen von Jodid in wäßrigen Lösungen halbquantitativ oder auch quantitativ bestimmen kann, ohne daß harte Vorbehandlungsmaßnahmen erforderlich sind, und ohne daß toxische Substanzen entstehen.

Gegenstand der Erfindung ist ein Verfahren zur halbquantitativen Bestimmung von Jodid in Harn, das dadurch gekennzeichnet ist, daß die Harnprobe mit Hilfe einer gereinigten Aktivkohle vorbehandelt. wird und man anschließend die Probelösung mit einem Chromogen und einer Persäurelösung versetzt und die Farbreaktion visuell oder photometrisch auswertet. Es ist vorteilhaft, wenn die gereinigte Aktivkohle in einer Extraktionssäule vorliegt.

Ein weiterer Gegenstand der Erfindung sind Mittel, d.h. Reagenzzusammenstellungen, zur Bestimmung von Jodid in Harn, die ein Chromogen, eine Persäurelösung und eine Aktivkohle enthaltende Extraktionssäule enthalten.

Es hat sich gezeigt, daß das erfindungsgemäße Verfahren weniger störanfällig und wesentlich schneller durchzuführen ist, als die bekannten Verfahren zur Jodidbestimmung im Harn. Es lassen sich noch Jodidkonentrationen bis zu 6 µg/dl neben einer hohen anionischen Matrixkonzentration nachweisen. In wäßrigen Lösungen ohne organische Matrix liegt die Nachweisgrenze für Jodid bei 0,2 µg/dl.

Für das Verfahren nach der Erfindung können als Chromogene alle in der Literatur bekannten Chromogene eingesetzt werden, die in Gegenwart von Persäuren mit Jodid als Katalysator oxidierbar sind. Geeignet sind vorzugsweise aromatische Amine wie o- und p-Phenylendiamin, Benzidin und Benzidinderivate, insbesondere Tetramethylbenzidin. Die Konzentration des Chromogens sollte im Bereich von 1 bis 5 mM, vorzugsweise bei etwa 2,5 mM liegen.

Als Persäurelösungen eignen sich z.B. wäßrige Lösungen von Peressigsäure, Perpropionsäure, Perbuttersäure, Perbenzoesäure usw., vorzugsweise eine wäßrige Peressigsäurelösung. Die Konzentration dieser Lösung sollte im Bereich von 1 bis 5 %, vorzugsweise bei etwa 2,5 % liegen.

Das erfindungsgemäße Mittel kann entweder aus zwei Lösungen, einer Persäurelösung und einer Lösung eines Chromogens bestehen oder neben der Persäurelösung kann das Chromogen, gegebenenfalls zusammen mit einer organischen Säure, in Form einer damit imprägnierten saugfähigen Matrix vorliegen.

Als saugfähige Matrices können alle verwendet werden, die üblicherweise für solche Schnelltests im Gebrauch sind. Am weitesten verbreitet ist die Verwendung von Filterpapier, jedoch können auch andere saugfähige Cellulose-, Glasfaser- oder Kunststoffprodukte eingesetzt werden. Die saugfähigen Träger, vorzugsweise Filterpapier, werden in an sich bekannter Weise mit Tränklösungen imprägniert, die das Chromogen und gegebenenfalls weitere notwendige Reagenzien enthalten. Die getränkten und getrockneten Papiere können zu quadratischen bzw. rechteckigen Zonen verarbeitet werden, die ihrerseits in bekannter Weise auf Kunststoffolien, Papier- oder Metallstreifen aufgeklebt bzw. aufgesiegelt werden können.

Die saugfähigen Träger können auch vor dem Imprägnieren in Streifenform auf ein Kunststoffband aufgebracht und nach dem Imprägnieren senkrecht zur Streifenrichtung in handliche Stäbchen geschnitten werden.

Um die Reaktion in einem optimalen pH-Bereich ablaufen zu lassen, enthält die Chromogenlösung gegebenenfalls Puffersubstanzen. Erfolgt die Bestimmung des Jodids mit Hilfe einer mit den erforderlichen Substanzen imprägnierten Matrix, ist die Anwesenheit von Puffersubstanzen ebenfalls von Vorteil.

Als Puffersubstanzen kommen solche in Frage, die einen pH-Wert im Bereich von 3 bis 7 einhalten können und die Nachweisreaktion nicht stören. Die einzusetzende Pufferkonzentration richtet sich nach dem pH-Wert der Probelösung und nach den dort eventuell vorliegenden freien Säuren oder Basen. Als Puffer sind die gebräuchlichen Salze wie Citrate, Phosphate, Borate usw. geeignet. Vorzugsweise werden feste organische Säuren wie Citronensäure, Malonsäure, Bemsteinsäure und deren Salze eingesetzt, insbesondere Citronensäure. Anstelle einer gebrauchsfertigen Pufferlösung können auch handelsübliche Pufferkonzentrate eingesetzt werden.

Im Falle der Verwendung von Tetramethylbenzidin als Chromogen wird der intensivste Farbwert bei einem pH-Wert von etwa 4 erreicht. Pufferlösungen mit pH-Werten bis zu 7 können ebenfalls eingesetzt werden, wobei sich dann etwas schlechtere Nachweisgrenzen ergeben.

Bei der Bestimmung von Jodid im Harn ist es wichtig, daß die Probelösung mit Hilfe von Aktivkohle vorbehandelt wird, insbesondere mit einer gereinigten Aktivkohle. Da schwefelhaltige Störsubstanzen auf den handelsüblichen Aktivkohlen die Farbreaktionen negativ beeinflussen, ist eine Aufreinigung in der Regel erforderlich. Dazu wird die Aktivkohle mit einer 1 N Salzsäure behandelt, anschließend erfolgt eine Konditionierung mit der bei der Bestimmung verwendeten Pufferlösung.

Da die Aktivkohle vorzugsweise in eine Säule gefüllt wird, sollte die Porengröße der Aktivkohle im Bereich von 50 bis 150 µm liegen. Bei kleineren Poren ist die Durchlässigkeit für wäßrige Lösungen eingeschränkt, bei größeren Poren verschlechtern sich die Adsorptionseigenschaften der Aktivkohle. Die Extraktionssäule enthält etwa 0,1 bis 0,4 g, vorzugsweise 0,2 g Aktivkohle für eine Bestimmung. Selbstverständlich kann die Bestimmung auch im sogenannten Batch-Verfahren durchgeführt werden.

Die Durchführung einer Bestimmung von Jodid im Harn mit Hilfe einer Aktivkohle enthaltenden Extraktionssäule erfolgt so, daß auf die konditionierte Säule eine mit Pufferlösung verdünnte Hamprobe gegeben wird, die dann verworfen wird. Anschließend wird die zu untersuchende verdünnte Hamprobe mit Hilfe der Säule gereinigt und in einem Meßbecher oder einer Küvette aufgefangen. Die so vorbehandelte Hamprobe wird mit der Chromogenlösung und danach mit der Persäurelösung versetzt und die Farbreaktion nach einer vorgegebenen Zeit von z.B. einer Minute mit Hilfe einer Farbskala oder photometrisch ausgewertet. Der Erfassungsbereich für Jodid nach dem erfindungsgemäßen Verfahren liegt bei 6 bis 100 µg/dl Jodid. Bei der Jodidbestimmung mit Hilfe eines saugfähigen Trägers ist der Erfassungsbereich bei 20-200 µg/dl Jodid.

Eine Probenvorbereitung mit Aktivkohle entfällt z.B. bei der Bestimmung von Jodid in Mineralwasser, Meerwasser, Speisesalz usw. In den Fällen, in denen eine störende Matrix entfemt werden muß oder bei Anwesenheit von Substanzen, die eine Eigenfarbe haben, und damit die photometrische oder visuelle Auswertung erschweren, z.B. bei bestimmten Nahrungs- und Futtermitteln, sowie bei Milch, ist eine Probenvorbereitung erforderlich.

### Beispiel 1:

### Reagenzlösungen

### Herstellung der Chromogenlösung:

600 mg Tetramethylbenzidin werden in 1000 ml Ethanol gelöst (2,5 mM).

Die gebrauchsfertige Pufferlösung (pH 4) enthält 11,7 g Citronensäure, 4,4 g Natriumhydroxid und 1,6 g Chlorwasserstoff in 1000 ml Wasser.

Die Persäurelösung enthält 2,5 g Peressigsäure in 100 ml Wasser.

### Beispiel 2

### Bestimmung von Jodid im Ham

### a) Probenvorbereitung

Auf eine Extraktionssäule, die 0,2 g Aktivkohle enthält, werden nacheinander 1 N Salzsäure und die in Beispiel 1 angegebene Pufferlösung gegeben. 2 ml einer mit Pufferlösung verdünnten Hamprobe (1 ml Ham, 4 ml Pufferlösung) werden auf die Säule gegeben und verworfen. Anschließend werden weitere 2 ml der verdünnten Hamprobe auf die Säule gegeben und in einem Meßbecher aufgefangen.

### b) Durchführung der Bestimmung

Die erhaltene Hamprobe wird mit 200 µl einer 2,5 mM Tetramethylbenzidinlösung und danach mit einem Tropfen einer 2,5 %igen Peressigsäurelösung versetzt. Es tritt entsprechend der Jodidkonzentration eine Farbreaktion von grün nach blau ein, die nach einer Minute mit einer Farbskala verglichen wird.

Für die photometrische Auswertung wird die Lösung in eine 10 mm-Küvette gefüllt und das Absorptionsmaximum bei 646 nm vermessen.

### Beispiel 3

### Herstellung eines Teststäbchens

In einer Mischung aus 500 ml Ethanol und 100 ml Wasser werden unter Rühren nacheinander 1,5 g Tetramethylbenzidin und 2,5 g Citronensäure aufgelöst.

Ein 6 mm breiter Papierstreifen, der auf einer 8 cm breiten weißen PVC-Folie als Träger aufgeklebt ist, wird mit der Chromogen enthaltenden Lösung getränkt (Auftragsmenge: 1 ml Tränklösung pro Meter Papier). Nach dem Trocknen wird die Folie in 6 mm breite Streifen (Teststäbchen) geschnitten.

Zu der nach Beispiel 2a) vorbereiteten Harnprobe wird ein Tropfen einer 2,5 %igen Peressigsäure getropft und umgeschüttelt. Das Teststäbchen wird sofort für etwa eine Sekunde eingetaucht. Überschüssige Lösung wird abgeschüttelt. Nach weiteren etwa 10 Sekunden wird mit einer Farbskala verglichen, Folgende Jodidkonzentrationen im Harn können durch unterschiedliche Blaufärbung der Reaktionszone unterschieden werden: 20, 50, 100, 200 µg/dl.

## Patentansprüche

1. Halbquantitatives Verfahren zur Bestimmung von Jodid in Harn in Konzentrationen bis zu 200 µg/dl Iodid, **dadurch gekennzeichnet, dass** man die Probelösung mit Hilfe einer gereinigten Aktivkohle vorbehandelt und anschließend mit einem Chromogen und einer Persäurelösung versetzt und die Farbreaktion visuell oder photometrisch auswertet.

2. Verfahren nach Anspruch 1, **dadurch gekennzeichnet, dass** man zur Bestimmung von Jodid im Harn die Probe mit Hilfe einer gereinigte Aktivkohle enthaltenden Extraktionssäule vorbehandelt.

## Claims

1. Semi-quantitative method for the determination of iodide in urea in concentrations of up to 200 µg/dl of iodide, **characterised in that** the sample solution is pre-treated with the aid of purified activated carbon, and a chromogen and a peracid solution are subsequently added, and the colour reaction is evaluated visually or photometrically.

2. Method according to Claim 1, **characterised in that**, for the determination of iodide in urea, the sample is pre-treated with the aid of an extraction column containing purified activated carbon.

## Revendications

1. Procédé semi-quantitatif pour la détermination d'iodure dans l'urée selon des concentrations de jusqu'à 200 µg/dl d'iodure, **caractérisé en ce que** la solution d'échantillon est prétraitée à l'aide de charbon actif purifié, et un chromogène et une solution de peracide sont ensuite ajoutés, et la réaction de coloration est évaluée visuellement ou photométriquement.

2. Procédé selon la revendication 1, **caractérisé en ce que**, pour la détermination d'iodure dans l'urée, l'échantillon est prétraité à l'aide d'une colonne d'extraction contenant du charbon actif purifié.
